# EUROPÄISCHE PATENTSCHRIFT

(11) **EP 3 184 550 B1**
(45) Veröffentlichungstag und Bekanntmachung des Hinweises auf die Patenterteilung: **22.08.2018**
(21) Anmeldenummer: 16205024.9
(22) Anmeldetag: 19.12.2016
(51) Int. Cl.: C08B 11/02, C08B 11/12, C08B 37/08

(54) **VERFAHREN ZUR STERILISATION VON WÄSSRIGEN POLYSACCHARIDLÖSUNGEN UND STERILE WÄSSRIGE POLYSACCHARIDLÖSUNGEN**
METHOD FOR THE STERILISATION OF AQUEOUS POLYSACCHARIDE SOLUTIONS AND STERILE AQUEOUS POLYSACCHARIDE SOLUTIONS
PROCÉDÉ DE STÉRILISATION DE SOLUTIONS DE POLYSACCHARIDE AQUEUSES ET SOLUTIONS DE POLYSACCHARIDE AQUEUSES STÉRILES

(30) Priorität: 22.12.2015 DE 102015226456
(43) Veröffentlichungstag der Anmeldung: 28.06.2017
(73) Patentinhaber: Heraeus Medical GmbH, 61273 Wehrheim (DE)
(72) Erfinder: Vogt, Sebastian, 99092 Erfurt (DE); Kluge, Thomas, 56179 Vallendar (DE)
(74) Vertreter: Heraeus IP

(56) Entgegenhaltungen:
- EP-A2- 0 228 698
- EP-A2- 1 734 073
- EP-B1- 2 596 812
- WO-A1-03/002159
- DE-A1-102005 017 845

## Beschreibung

Gegenstand der Erfindung ist ein Verfahren zur Sterilisation von wässrigen Polysaccharidlösungen sowie die durch dieses Verfahren hergestellte Polysaccharidlösungen.

Arthrose (*Arthrosis deformans*) ist eine weit verbreitete degenerative Erkrankung der Gelenke. Dabei kommt es zu einer Schädigung (Erosion) der Knorpeloberflächen, der Ablösung von Knorpelpartikeln und durch Knorpelpartikel verursachte Entzündungen der Synovialmembran. Bei leichter und mittelschwerer Arthrose wird seit einigen Jahren versucht, durch eine intraartikuläre Injektion von Hyaluronsäure (Viscosupplementation) die Schmerzsituation der Patienten zu verbessern und gleichzeitig das Fortschreiten der Arthrose zu vermindern.

Hyaluronsäure ist ein natürlicher Bestandteil der Gelenkflüssigkeit (Synovialflüssigkeit). Die Hyaluronsäure wirkt in der Gelenkflüssigkeit als Schmiermittel. Besonders vorteilhaft ist es, dass wässrige Hyaluronsäurelösungen viskoelastisch sind. Dadurch ergeben sich sehr gute Schmier- und Gleiteigenschaften.

Auf Grund der vorteilhaften Schmiereigenschaften werden wässrige Hyaluronsäurelösungen seit ungefähr zwei Jahrzehnten zur Viscosupplementation verwendet. Stand der Technik ist gegenwärtig die Verwendung von fermentativ hergestellter Hyaluronsäure, welche in Form einer sterilen, wässrigen Lösung der Hyaluronsäure verwendet wird. Daneben ist auch die Verwendung von in Wasser löslichen Cellulosederivaten, wie Carboxymethylcellulose und Methylcellulose, von Stärkederivaten, wie Hydroxyethylstärke, zur Viskosupplementation grundsätzlich möglich.

Wässrige Hyaluronsäurelösungen werden bisher hauptsächlich durch Gammabestrahlung sterilisiert. Dabei sind Dosen von gleich/größer 25 kGy üblich. Diese Sterilisation wird an endverpackter Hyaluronsäurelösung vorgenommen.

Die Gammabestrahlung ist jedoch mit gravierenden Nachteilen verbunden. Neben einem Abbau der Polymerketten, durch den - abhängig von der Dosis der Gammastrahlung - mehr oder weniger niedermolekulare Zerfallsprodukte entstehen, können auch Nebenreaktionen auftreten, die zu Verfärbungen der Hyaluronsäurelösungen führen. Ein weiterer Nachteil bei der Verwendung von Gammastrahlung besteht darin, dass die üblichen Gammaquellen ein kugelförmiges Strahlenfeld haben. Dadurch kann die einwirkende Dosis in Abhängigkeit von der Position des zu sterilisierenden Gegenstandes variieren. Es erfolgt ein ungleichmäßiger Polymerabbau, womit Inhomogenitäten bei der finalen Viskosität möglich sind. Eine reproduzierbare Endviskosität der sterilsierten Hyaluronsäurelösungen ist kaum zu erreichen. Die Gammastrahlung kann ferner auch zur Versprödung der Packmittel, meistens Einwegspritzen aus Kunststoff, führen.

Ähnliche Nachteile hat die Dampfsterilisation von wässrigen Hyaluronsäurelösungen, die zu Schädigungen an der Hyaluronsäure und an den Kunststoffpackmitteln führen kann.

Eine Sterilfiltration von wässrigen Hyaluronsäurelösungen ist auf Grund der recht hohen Viskosität der Lösungen praktisch nicht oder nur sehr aufwendig möglich. Außerdem werden bei der Sterilfiltration nur mikrobielle Lebensformen ab einer bestimmten Größe entfernt. Viren können durch Sterilfiltration nicht entfernt oder inaktiviert werden.

Neben diesen physikalischen Sterilisationsverfahren werden oft auch chemische Verbindungen zur Sterilisation von Medizinprodukten eingesetzt.

Dazu gehören zum Beispiel Formaldehyd, Glutardialdehyd, o-Phthaldialdehyd. Die Sterilisation mit Aldehyden ist mit dem Nachteil verbunden, dass diese nach der Sterilisation wieder entfernt werden müssen, um bei der Anwendung am Menschen Schädigungen zu vermeiden. Bei endverpackten wässrigen Hyaluronsäurelösungen ist dadurch eine Sterilisation mit Aldehyden ausgeschlossen. Aldehyde können aus endverpackten Hyaluronsäurelösungen nicht wieder entfernt werden.

Sehr wirksame Sterilisationsmittel stellen oxidierende Agenzien, wie Wasserstoffperoxid, Perameisensäure, Peressigsäure, Hypochlorid und Hypochlorid abspaltende Substanzen, wie Chloramin T2 oder Trichlorisocyanursäure, dar. Nachteilig an diesen Agenzien ist, dass diese einen erheblichen oxidativen Abbau der gelösten Hyaluronsäure verursachen. Weiterhin können nicht umgesetzte Reste der oxidierenden Agenzien in der endverpackten Hyaluronsäurelösung verbleiben, die gegebenenfalls lokaltoxisch sein können.
Aus der pharmazeutischen Industrie ist bekannt, dass wässrige Proteinlösungen, wie zum Beispiel Impfseren, gegenüber oxidierenden Sterilisationsmitteln und verschiedenen physikalischen Sterilisationsverfahren, zum Beispiel der Sterilisation mit Gammastrahlung, sehr empfindlich sind. Aus diesem Grund werden diese wässrigen Proteinlösungen zuerst sterilfiltriert und dann zur Inaktivierung von Viren mit geringen Mengen an β-Propiolacton versetzt. β-Propiolacton acyliert die Aminogruppen der DNA/RNA oder von Proteinen der Viren. Das als Lösungsmittel enthaltene Wasser ist in der Lage, das β-Propiolacton langsam zu zersetzen, so dass in den wässrigen Proteinlösungen nach kurzer Zeit kein aktives β-Propiolacton mehr enthalten ist. Bisher ist bekannt, dass gasförmiges β-Propiolacton Endoporen irreversibel inaktivieren kann (R. K. Hoffmann, B. Warshowsky: Beta-Propiolactone Vapor as a Disinfectant. Appl. Microbiol. 1958 Sept.; 6(5): 358-362). Weiterhin ist bekannt, dass β-Propiolacton in nicht wässrigen organischen Monomeren/Monomergemischen und pastenförmigen Zementen, die organische Monomere enthalten, Endosporen inaktiviert (EP 2 596 812 B1).
Neben den vegetativen Formen der Mikroorganismen gibt es jedoch auch generative Formen, wie zum Beispiel Endosporen. Sie stellen generative Überdauerungsformen von Mikroorganismen dar und werden von Gram-positiven Bakterien, besonders der Gattungen Bacillus und Clostridium, gebildet, um ungünstige Lebensbedingungen überdauern zu können. Endosporen haben im Ruhezustand keinen aktiven Stoffwechsel und besitzen eine mehrschichtige Sporenhülle, die den Sporen-Kern vor Einwirkung von Chemikalien und anderen Umwelteinflüssen weitgehend schützt.

Dadurch sind Endosporen extrem widerstandsfähig gegenüber der Einwirkung von Hitze und von Chemikalien (Borick, P. M.: Chemical sterilizers. Adv. Appl. Microbiol. 10 (1968) 291-312; Gould, G. W.: Recent advances in the understanding of resistance and dormacy in bacterial spores. J. Appl. Bacteriol. 42 (1977) 297-309; Gould, G. W.: Mechanisms of resistance and dormancy. p. 173-209. In Hurst, A. and Gould, G. W. (ed.), The bacterial spore. vol. 2 Academic Press, Inc. New York, 1983). Endosporen werden aufgrund ihrer hohen Widerstandsfähigkeit als Bioindikatoren für die Validierung und die Wirksamkeitskontrolle von Sterilisationsprozessen eingesetzt. Es wird dabei davon ausgegangen, dass eine Inaktivierung der Endosporen eine Abtötung aller vegetativen mikrobiellen Lebensformen abbildet. Endosporen von Gram-positiven Bakterien gehören der internationalen Resistenzstufe III an. Zu den Resistenzstufen I gehören nichtsporenbildende Bakterien und vegetative Formen von Sporenbildnern und zur Resistenzstufe II zählen Sporen, die in strömenden Wasserdampf bei 105 °C innerhalb weniger Minuten abgetötet werden. Bei einer Sterilisation müssen nach DAB (Deutsches Arzneimittelbuch) 2008 alle Mikroorganismen der Resistenzstufen I-III abgetötet beziehungsweise irreversibel inaktiviert werden.
Die Aufgabe der Erfindung besteht in der Entwicklung eines Verfahrens zur Sterilisation von wässrigen Polysaccharidlösungen. Verfahren zur Herstellung von sterilen Polysaccharidlösungen sind beispielsweise aus EP 1 734 073 bekannt. Dieses Verfahren soll eine Sterilisation ermöglichen, ohne dass ein signifikanter Polymerabbau der gelösten Polysaccharide erfolgt.
Außerdem soll das zu entwickelnde Sterilisationsverfahren geeignet sein, in Endverpackungen gelagerte wässrige Polysaccharidlösungen, einschließlich der Innenwände der Packmittel zu sterilisieren, die mit der wässrigen Polysaccharidlösung in Berührung stehen. Das zu entwickelnde Sterilisationsverfahren soll Mikroorganismen der Resistenzstufe III sicher inaktivieren.
Im Rahmen der Erfindung wird unter Sterilität ein von lebensfähigen Mikroorganismen befreiter Zustand entsprechend der EN 556-1:2001 bezeichnet.

Die Aufgabe der Erfindung wird gemäß Anspruch 1 durch ein Verfahren zur Sterilisation von wässrigen Polysaccharidlösungen gelöst. Erfindungsgemäß wird zu einer wässrigen Lösung eines Polysaccharids in Gegenwart eines Puffers ein β-Lacton gegeben und die Mischung bei einer Temperatur von 4°C bis 40 °C über einen Zeitraum von mindestens 24 Stunden gelagert.

Bevorzugt ist ein Verfahren gekennzeichnet durch
a) Lösen mindestens eines Polysaccharids in Wasser,
b) Zugabe von mindestens 0,5 Gew.-% β-Lacton zu der unter a) hergestellten wässrigen Polysaccharidlösung, und
c) Lagern des Gemisch bei einer Temperatur von 4°C bis 40 °C über einen Zeitraum von mindestens 24 Stunden, wobei
ein Puffersystem in der wässrigen Lösung enthalten ist, dessen Pufferkapazität so groß ist, dass die durch Hydrolyse des β-Lactons entstehende 3-Hydroxypropansäure so abgepuffert wird, dass der pH-Wert der sterilisierten wässrigen Polysaccharidlösung gleich der unsterilisierten Polysaccharidlösung ist.

Die Erfindung wird ferner gemäß Anspruch 14 durch eine sterile Polysaccharidlösung hergestellt nach einem der obigen Verfahren gelöst.

Überraschend wurde gefunden, dass mit Hilfe eines Verfahrens gemäß Anspruch 1 eine wässrige Polysaccharidlösung sterilisiert werden kann, wobei nur ein minimaler Polymerabbau stattfindet. Da die Viskosität ein Maß für das Molekulargewicht der gelösten Polysaccharide ist, bedeutet dies, dass das Verhältnis der reduzierten spezifischen Viskosität der sterilisierten Polysaccharidlösung zur reduzierten spezifischen Viskosität der unsterilen Polysaccharidlösung bei größer gleich 0,8 gehalten werden kann. Bei der Bestimmung dieses Verhältnisses ist es unerheblich, ob hier die reduzierte Viskosität, die dynamische Viskosität oder die kinematische Viskosität ermittelt wird. Bei wässrigen Polysaccharidlösungen, die einer Gelpermeationschromatographie zugänglich sind, kann das Verhältnis der zahlenmittleren Molmasse des sterilisierten Polysacharids zur zahlenmittleren Molmasse des unsterilisierten Polysaccharids größer gleich 0,8 gehalten werden.

Weiterhin verursacht das erfindungsgemäße Sterilisationsverfahren keine Verfärbungen infolge von Nebenreaktionen des Sterilisationsprozesses.

Mit Hilfe des erfindungsgemäßen Verfahrens werden in wässrigen Polysaccharidlösungen suspendierte Endosporen der Resistenzstufe III durch Einwirkung von β-Lacton inaktiviert. Dabei ist ein Puffer in den Polysaccharidlösungen enthalten, der eine solche Kapazität hat, dass die durch Hydrolyse des β-Lactons frei werdende 3-Hydroxypropionsäure abgepuffert wird, so dass der pH-Wert der sterilisierten Polysaccharidlösungen gleich dem pH-Wert der zuvor unsterilen Polysaccharidlösungen besitzt. Bevorzugt werden hierbei Puffer in derselben Menge wie das β-Lacton eingesetzt.

Die Erfindung beruht weiter auf dem überraschenden Befund, dass mit β-Lacton eine Sterilisation von wässrigen Polysaccharidlösungen in Gegenwart eines Puffers vorgenommen werden kann, ohne dass bei der Sterilisation ein signifikanter Polymerabbau der Polysaccharide erfolgt. Der besondere Vorteil der erfindungsgemäßen Sterilisation besteht darin, dass die Viskosität der Polysaccharidlösungen vor und nach der Sterilisation gleich bleibt. Das bedeutet, für die Verwendung von wässrigen Polysaccharidlösungen zur Viscosupplementation kann exakt die Viskosität der Lösungen vor der Sterilisation eingestellt werden, ohne dass sich die Viskosität durch den Sterilisationsprozess verändert. Das hat zur Folge, dass für die Viscosupplementation hinsichtlich der Viskosität und der sonstigen rheologischen Eigenschaften exakt eingestellte sterile Polysaccharidlösungen bereitgestellt werden können. Ein weiterer Vorteil besteht darin, dass es zu keinen Verfärbungen der wässrigen Polysaccharidlösungen infolge der Sterilisation kommt.

Polysaccharide sind Kohlenhydrate, in denen eine große Anzahl (mindestens zehn) Monosaccharide über eine glycosidische Bindung verbunden sind. Wasserlösliche Polysaccharide im Sinne der vorliegenden Erfindung sind Polysaccharide, die aus Monosacchariden, Disacchariden, weiteren Oligomeren davon oder Derivaten bestehen und in der gleichen Art miteinander verknüpft sind

Erfindungsgemäß handelt es sich bei den Polysacchariden um natürliche Polysaccharide und künstliche Polysaccharide. Unter Derivaten sind erfindungsgemäß Salze, Ether, Ester der Säuren oder Ester, insbesondere Alkalimetallsalze, besonders Natrium- und Kaliumsalze zu verstehen. Beispiele sind Alginsäure, Natriumalginat, Hyaluronsäure, das Natriumsalz der Hyaluronsäure, Carboxymethylcellulose, das Natriumsalz der Carboxymethylcellulose, Hydroxyethylcellulose, Celluloseether, Stärke, Stärkeether, Guar, Chitin, Chitosan.

Bevorzugt ist das Polysaccharid aus der Gruppe ausgewählt, die aus dem Natriumsalz der Hyaluronsäure, dem Natriumsalz der Carboxymethylcellulose, Hydroxyethylcellulose, Hydroxyethylstärke, Methylcellulose und oxidierter Cellulose besteht.

Erfindungsgemäß kann es sich bei den Polysacchariden auch um Mischungen der genannten Polysaccharide handeln.

Die Polysaccharide werden bevorzugt in einer Menge von mindestens 0,5 Gew.-% eingesetzt. Sie werden ferner bevorzugt in einer Menge von maximal 5 Gew.-% eingesetzt. Besonders bevorzugt werden Polysaccharide in einer Menge von 1 bis 2 Gew.-% eingesetzt. Die Menge bezieht sich jeweils auf die Menge an Polysaccharid in Wasser.

Bei β-Lacton es sich um eine Verbindung (a1) handeln, die durch die allgemeine Formel (I) 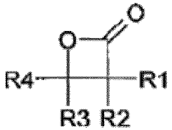 dargestellt ist. In dieser Formel können R1, R2, R3 und R4 unabhängig voneinander für H, einen substituierten oder unsubstituierten Alkylrest, einen Halogenrest, einen Nitrorest oder einen Cyanorest stehen.

Die Stereochemie der Verbindungen (a1) ist nicht weiter eingeschränkt. Vorzugsweise können im Rahmen der Erfindung als Verbindung (I) alle Isomere, die unter die allgemeine Formel (I) fallen, unabhängig von ihrer genauen Konfiguration eingesetzt werden.

Die Alkylreste können unabhängig voneinander substituierte oder unsubstituierte Alkylreste sein. Der wenigstens eine Substituent eines substituierten Alkylrestes ist vorzugsweise aus der Gruppe ausgewählt, die aus Halogenresten, Nitroresten und Cyanoresten besteht.

Die Alkylreste können unabhängig voneinander gesättigte oder ungesättigte Alkylreste sein. Vorzugsweise weist ein ungesättigter Alkylrest wenigstens eine Kohlenstoff-Kohlenstoff-Doppelbindung auf.

Die Alkylreste können unabhängig voneinander verzweigte oder unverzweigte Alkylreste sein. Als Alkylreste R1, R2, R3 und R4 bevorzugt sind unverzweigte Alkylreste.

Die Alkylreste weisen unabhängig voneinander vorzugsweise eine Länge der Hauptkette im Bereich von 1 - 4 Kohlenstoffatomen, mehr bevorzugt eine Länge der Hauptkette im Bereich von 1 - 2 Kohlenstoffatomen und noch mehr bevorzugt ein Kohlenstoffatom auf.

Als Halogenreste kommen in der allgemeinen Formel (I) vorzugsweise Fluorreste, Chlorreste und Bromreste in Betracht. Diese Reste können jeweils unabhängig voneinander für einen oder mehrere der Reste R1, R2 R3 und R4 stehen.

Gemäß einer bevorzugten Ausführungsform stehen die Reste R1, R2, R3 und R4 jeweils für H.

Gemäß einer weiteren bevorzugten Ausführungsform steht der Rest R1 für einen Methylrest und stehen die Reste R2, R3 und R4 für H.

Gemäß einer weiteren bevorzugten Ausführungsform stehen die Reste R1, R2 und R3 für H und steht der Rest R4 für einen Methylrest.

Gemäß noch einer weiteren Ausführungsform stehen die Reste R1 und R3 für H und die Reste R2 und R4 für einen Methylrest.

Gemäß einer besonders bevorzugten Ausführungsform handelt es sich bei der Verbindung (a1) um β-Propiolacton (CAS-Nummer 57-57-8).

Bei dem β-Lacton kann es sich auch um eine Verbindung (a2) handeln, die ein Dimer aus einer der Verbindungen (a1) ist. Die Stereochemie der Verbindungen (a2) ist nicht weiter eingeschränkt.

Erfindungsgemäß ist als Puffer Phosphatpuffer mit einem pH-Wert von 7,0-8,0 bevorzugt, wobei besonders ein Phosphatpuffer mit einem pH-Wert von 7,4 bevorzugt ist.

Zur Herstellung des Phosphatpuffers sind insbesondere die Kombination von Kaliumhydrogenphosphat und Dinatriumhydrogenphosphat, die Kombination Natriumhydropgenphosphat und Dinatriumhydrogenphosphat, die Kombination von Dinatriumphosphat und Phosphorsäure und die Kombination Trinatriumphosphat mit Natriumhydrogenphoosphat und die Kombination Trinatriumphosphat und Phosphorsäure geeignet.

Die gesamten Komponenten des Phosphat-Puffers sind bevorzugt in einer Konzentration von größer gleich 0,5 Gew.-% in der wässrigen Polysaccharidlösung gelöst.

Erfindungsgemäß ist, dass die durch die Hydrolyse des β-Propiolactons entstehende 3-Hydroxypropionsäure ein Bestandteil des Puffers ist.

Es ist erfindungsgemäß, dass die 3-Hydroxypropansäure und das Natriumsalz der Hyaluronsäure oder 3-Hydroxypropansäure und das Natriumsalz der Carboxymethylcellulose einen Puffer bilden.

Wesentlich für die Erfindung ist, dass die Sterilisation der wässrigen Polysaccharidlösung in der primären Verpackung erfolgt. Dadurch ist kein zusätzlicher Aufwand, wie Gammabestrahlung oder Bestrahlung mit Elektronen oder eine Autoklavierung notwendig. Es können somit erhebliche Kosten in der Herstellung eingespart werden.

Wichtig ist für die erfolgreiche Sterilisation, dass die Vermischung der wässrigen Polysaccharidlösung mit β-Propiolacton unmittelbar vor der Befüllung des primären Packmittels mit der wässrigen Polysaccharidlösung erfolgt. Dadurch wird sichergestellt, dass genügend Sterilisationsagens sowohl zur Sterilisation der Polysaccharidlösung als auch der Innenwände des primären Packmittels zur Verfügung steht. Wenn dagegen die Polysaccharidlösung längere Zeit vor der Abfüllung in das primäre Packmittel mit β-Propiolacton vermischt wird, kann es je nach Temperatur und der vergangen Zeit bis zur Abfüllung in das primäre Packmittel zu einem Verlust an β-Propiolacton infolge der Hydrolyse zur 3-Hydroxypropionsäure gekommen sein. Dadurch kann die insbesondere die Sterilisation der Innenwände des primären Packmittels in Frage gestellt sein.

Erfindungsgemäß ist eine sterile wässrige Polysaccharidlösung, die nach dem erfindungsgemäßen Verfahren hergestellt wird. Diese sterile Polysaccharidlösung ist dadurch charakterisiert, dass die wässrige Polysaccharidlösung mindestens ein in Wasser zu mindestens teilweise lösliches Polysaccharid, Wasser und 3-Hydroxypropionsäure und ein Puffersystem enthält.

Weiterhin ist es erfindungsgemäß, dass die sterile wässrige Polysaccharidlösung ein Puffersystem aus Natrium-Ionen, Dihydrogenphosphat-Ionen, Hydrogenphosphatlonen, Phosphat-Ionen, 3-Hydroxypropionsäure und 3-Hydroxypropionat enthält. Gegebenenfalls können auch Kalium-Ionen enthalten sein.

Erfindungsgemäß ist, dass das Verhältnis der reduzierten Viskosität der sterilisierten Polysaccharidlösung zur reduzierten Viskosität der nicht sterilisierten Polysaccharidlösung größer 0,8 ist.

Erfindungsgemäß ist auch die Verwendung der sterilen, wässrigen Polysaccharidlösung als Mittel zur Viscosupplementierung und als pharmazeutischer Wirkstoffträger in der Humanmedizin und in der Veterinärmedizin.

Die Erfindung wird durch nachstehende Beispiele erläutert, ohne dass diese jedoch die Erfindung beschränken.

### Beispiele

Es wurden folgende Polysaccharide bzw. Polysacchariderivate für die nachfolgend beschriebenen Versuche eingesetzt:
Natriumsalz der Hyaluronsäure (Mₙ ∼ 1,3 Millionen Dalton, Fa. Kraeber GmbH),
Natriumsalz der Carboxymethylcellulose (Mₙ ∼ 90000 Dalton, Sigma-Aldrich),
Methylcellulose (SM-4000, Shin-Etsu-Chemical Co.),
Hydroxyethylcellulose (60SH-4000, Shin-Etsu-Chemical. Co)

Es wurde ein Phosphat-Puffer mit einem pH-Wert von 7,4 hergestellt. Dazu wurden 1,65 g Kaliumhydrogenphosphat und 9,71 g Dinatriumhydrogenphosphat-Dihydrat in einem Liter destillierten Wasser gelöst.

### Beispiel 1

Es wurden mit jeweils 30 ml Phosphat-Puffer (pH-Wert von 7,4) Lösungen der Polysaccharide hergestellt.

| Polysaccharid | Konzentration des Polysaccharids im Phosphat-Puffer [Gew.-%] |
|---|---|
| Natriumsalz der Hyaluronsäure | 0,25 |
| Natriumsalz der Carboxymethylcellulose | 2,00 |
| Methylcellulose (SM-4000) | 1,00 |
| Hydroxyethylcellulose (60SH-4000) | 1,00 |

Zu jeweils 5 ml der Polysaccharidlösungen in einem sterilen 25 ml Plastiktube wurden 10⁶ KBE einer Sporensuspension von *Bacillus atropheus* gegeben. Danach wurden die Sporen mit Hilfe eines Vortex-Mischgerätes homogen suspendiert. Anschließend wurden 0,5 Gew.-%, 1,0 Gew.-% und 2,0 % β-Propiolacton zu jeweils 5 ml der zuvor mit Sporen vermischten Polysaccharid-Lösung zugesetzt. Danach wurde nochmals mit einem Vortex-Mischgerät homogenisiert. Als Positiv-Kontrolle wurden nicht mit β-Propiolacton behandelte Polysaccharidlösungen mitgeführt. Nach 48 Stunden Lagerung der Polysaccharidlösungen bei Raumtemperatur erfolgte eine Prüfung auf Sterilität gemäß DIN EN ISP 11737, Teil 2. Es wurden dazu Doppelbestimmungen ausgeführt.

| Polysaccharid | Konzentration des Polysaccharids im Phosphat-Puffer [Gew.-%] | Ergebnisse der Sterilprüfung | | | |
|---|---|---|---|---|---|
| | | Konzentration β-Propiolacton [Gew.-%] | | | |
| | | 0,0 (Positiv-Kontrolle) | 0,5 | 1,0 | 2,0 |
| Natriumsalz der Hyaluronsäure | 0,25 | +/+ | +/- | -/- | -/- |
| Natriumsalz der Carboxymethylc ellulose | 2,00 | +/+ | -/- | -/- | -/- |
| Methylcellulose (SM-4000) | 1,00 | +/+ | -/- | -/- | -/- |
| Hydroxyethylcellulose (60SH-4000) | 1,00 | +/+ | -/- | -/- | -/- |

| | | | | | |
|---|---|---|---|---|---|
| (+) Wachstum (-) kein Wachstum) | | | | | |

Die mit β-Propiolacton sterislierten Polysaccharidlösungen zeigten keinerlei Verfärbung gegenüber den als Positiv-Kontrolle mitgeführten unbehandelten Polysaccharidlösungen.

### Beispiel 2

Es wurden folgende Polysaccharide für die nachfolgend beschriebenen Versuche eingesetzt:
NaHya 1: Natriumsalz der Hyaluronsäure (Mₙ ∼ 1,0 Millionen Dalton, Sigma-Aldrich)
NaHya 2: Natriumsalz der Hyaluronsäure (Mₙ ∼ 1,3 Millionen Dalton, Fa. Kraeber GmbH)
NaHya 3:Natriumsalz der Hyaluronsäure (Mₙ ∼ 200.000 Dalton, mit Chloridoxid degradierte Hyaluronsäure)

Die Hyaluronsäuren wurden zu jeweils 1 Gew.-% in Phosphatpuffer pH 7,4 gelöst. Es wurden jeweils 5 ml der Hyaluronsäurelösungen mit 100 µl β-Propiolacton vermischt und bei Raumtemperatur 48 Stunden gelagert. Parallel wurden nicht mit β-Propiolacton behandelte Hyaluronsäurelösungen zur Kontrolle mit eingelagert. Als zusätzlicher Vergleich wurden nicht mit β-Propiolacton behandelte Hylauronsäurelösungen gammasterilisiert. Die Gammasterilisation der Hyaluronsäurelösungen wurde von der Firma BBF Sterilisationsservice GmbH mit einer Co⁶⁰-Quelle mit einer Dosis von 25,2 kGy vorgenommen.

Die Hyaluronsäurelösungen hatten im unsterilen Zustand eine pH-Wert von 7,4 und nach der Sterilisation mit β-Propiolacton ebenfalls einen pH-Wert von 7,4. Die mit β-Propiolacton sterilisierten Hyaluronsäurelösungen zeigten keinerlei Farbveränderungen gegenüber den unsterilen Hyaluronsäurelösungen. Sowohl die unsterilen Hyaluronsäurelösungen als auch die mit β-Propiolacton sterilisierten Hyaluronsäurelösungen waren sichtklar und farblos. Dagegen zeigten die durch Gammabestrahlung sterilisierten Hyaluronsäurelösungen eine leichte Gelbverfärbung.

Die Molmassen und die Molmassenverteilung der Hyaluronsäureproben wurden mit Hilfe der Gelpermeationschromatographie unter Verwendung von Pullulan-Standards bestimmt. Als Detektor fand ein Brechungsindexdetektor (RI-Detektor) Verwendung. Die Messungen erfolgten als Doppelbestimmungen mit einer GPC-Anlage der Firma Jasco.

| Polysaccharidlösung | Zustand | Mn RI | Mw RI | D RI |
|---|---|---|---|---|
| NaHya 1 | unsteril | 923.300 | 5.714.500 | 6,39 |
| | β-Propiolacton steril isiert | 1.078.900 | 6.493.300 | 6,60 |
| | Gamma-sterilisiert | 11.950 | 8.791 | 1,60 |
| NaHya 2 | unsteril | 1.283.600 | 6.577.900 | 5,23 |
| | β-Propiolacton steril isiert | 1.325.400 | 6.260.600 | 4,73 |
| | Gamma-sterilisiert | 10.250 | 7.587 | 1,56 |
| NaHya 3 | unsteril | 218.400 | 479.000 | 2,24 |
| | β-Propiolacton steril isiert | 216.100 | 485.200 | 2,25 |
| | Gamma-sterilisiert | 10.132 | 7.475 | 1,57 |

| | | | | |
|---|---|---|---|---|
| Mₙ: zahlenmittlere Molmasse M_{w}: gewichtsmittlere Molmasse D: Polydisperisität | | | | |

Die Sterilisation der Polysaccharidlösungen mit β-Propiolacton bewirkte keinen Polymerabbau. Im Vergleich dazu führte die Gammasterilisation zu einem signifikanten Polymerabbau.

### Beispiel 3

Es wurden folgende Polysacchariderivate für die nachfolgend beschriebenen Versuche eingesetzt:
Natriumsalz der Carboxymethylcellulose (M_{w} ∼ 90000 Dalton, Sigma-Aldrich),
Hydroxyethylcellulose (60SH-4000, Shin-Etsu-Chemical. Co)

Es wurden mit jeweils 30 ml Phosphat-Puffer (pH-Wert von 7,4) Lösungen der Polysaccharide hergestellt.

| Polysaccharid | Konzentration des Polysaccharids im Phosphat-Puffer [Gew.-%] |
|---|---|
| Natriumsalz der Carboxymethylcellulose | 2,00 |
| Methylcellulose (60SH-4000) | 1,00 |

In jeweils 5 ml der Polysaccharidlösungen wurden 100 mg β-Propiolacton gegeben und nach erfolgter Vermischung 48 Stunden bei Raumtemperatur gelagert. Unbehandelte Polysaccharidlösungen wurden als Kontrolle mitgeführt. Für die viskosimetrische Vermessung der Lösungen wurden diese auf eine Polysaccharidkonzentration von 0,2 Gew.-% und auf 0,4 Gew.-% verdünnt. Die durch Gammabestrahlung sterilisierten Polysaccharidlösungen wurden auf Grund des hohen Polymerabbaus unverdünnt vermessen.

Die mit β-Propiolacton sterilisierten Polysaccharidlkösungen zeigten keine Farbveränderungen gegenüber den unsterilen Polysaccharidlösungen.

Die verdünnten Polysaccharidlösungen wurden mit einem Ubbelohde-Viskosimeter (Kapillare I, K = 0.010257) bei 25°C gemessen (5-fach Messung).

| Probe | Zustand | Konzentration | kinematische Viskosität |
|---|---|---|---|
| | | [Gew.-%] % | [mm²/s] |
| Carboxymethylcellulose NatriumSalz | unsteril | 0,4 | 2,311 ± 0,0004 |
| | β-Propiolacton sterilisiert | 0,4 | 2,317 ± 0,002 |
| | Gamma-sterilisiert | 2,0 | 1,503 ± 0,001 |
| Hydroxyethylcellulose (60SH-4000) | unsteril | 0,2 | 3,659 ± 0,003 |
| | β-Propiolacton sterilisiert | 0,2 | 3,590 ± 0,003 |
| | Gammasterilisiert | 1,0 | 1,169 ± 0,001 |

Anmerkung: Es wurden sichtklare Methylcelluloselösungen eingesetzt. Es kann jedoch nicht ausgeschlossen werden, dass transparente Gelpartikel mit in der Methylcelluloselösung enthalten waren. Dadurch kann die geringfügig höhere kinematische Viskosität der sterilisierten Methylcelluloselösung gegenüber der nicht sterilisierten Methylcelluloselösung erklärt werden. Wahrscheinlich war ein Gelpartikel mit in der sterilisierten Methylcelluloselösung.

Die kinematische Viskosität der mit β-Propiolacton sterilisierten Polysaccharidlösungen ist praktisch gleich der kinematischen Viskosität der unsterilen Polysaccharidlösungen. Bei der Sterilisation der Polysaccharidlösungen mit β-Propiolacton findet kein Polymerabbau statt. Dagegen führt die Gammasterilisation zu einem deutlichen Polymerabbau wie der Vergleich der kinematischen Viskosität der Gamma-sterilisierten Polysaccharidlösungen mit der kinematischen Viskosität der unsterilen Polysaccharidlösungen zeigt.

## Patentansprüche

1. Verfahren zur Sterilisation von wässrigen Polysaccharidlösungen,
**dadurch gekennzeichnet, dass**
zu einer wässrigen Lösung eines Polysaccharids in Gegenwart eines Puffers ein β-Lacton gegeben wird und die Mischung bei einer Temperatur von 4°C bis 40 °C über einen Zeitraum von mindestens 24 Stunden gelagert wird.

2. Verfahren nach Anspruch 1, **dadurch gekennzeichnet, dass**
das β-Lacton ausgewählt ist aus Verbindungen (a1), die durch die allgemeine Formel (I) 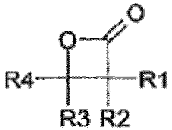 dargestellt sind, wobei R₁, R2, R3 und R4 unabhängig voneinander für H, einen substituierten oder unsubstituierten Alkylrest, einen Halogenrest, einen Nitrorest oder einen Cyanorest stehen, und Verbindungen (a2), die aus der Gruppe ausgewählt sind, die aus Dimeren der Verbindungen (a1) besteht.

3. Verfahren nach Anspruch 2, **dadurch gekennzeichnet, dass** das β-Lacton β-Propiolacton (CAS-Nummer 57-57-8).

4. Verfahren nach einem der Ansprüche 1 bis 3,
**dadurch gekennzeichnet, dass**
das β-Lacton in einer Menge von größer gleich 0,5-Gew.%, bezogen auf die Menge der wässrigen Polysaccharidlösung zugegeben wird.

5. Verfahren nach einem der Ansprüche 1 bis 4, **dadurch gekennzeichnet, dass**
das Polysaccharid aus der Gruppe ausgewählt ist, die aus dem Natriumsalz der Hyaluronsäure, dem Natriumsalz der Carboxymethylcellulose, Hydroxyethylcellulose, Hydroxyethylstärke, Methylcellulose und oxidierter Cellulose besteht.

6. Verfahren nach einem der Ansprüche 1 bis 5, **dadurch gekennzeichnet, dass** als Puffersystem Phosphatpuffer mit einem pH-Wert von 7,0-8,0, bevorzugt mit einem pH-Wert von 7,4 eingesetzt werden.

7. Verfahren nach Anspruch 6, **dadurch gekennzeichnet, dass**
der Phosphatpuffer aus der Gruppe ausgewählt ist, die aus der Kombination von Kaliumhydrogenphosphat und Dinatriumhydrogenphosphat, der Kombination von Natriumhydropgenphosphat und Dinatriumhydrogenphosphat, der Kombination von Dinatriumphosphat und Phosphorsäure, der Kombination Trinatriumphosphat mit Natriumhydrogenphoosphat und der Kombination Trinatriumphosphat und Phosphorsäure besteht.

8. Verfahren nach den Ansprüchen 1 bis 3, und 6 **dadurch gekennzeichnet, dass** die durch die Hydrolyse des β-Propiolactons entstehende 3-Hydroxypropionsäure ein Bestandteil des Puffersystems ist.

9. Verfahren nach den Ansprüchen 1 bis 3 **dadurch gekennzeichnet, dass** 3-Hydroxypropansäure und das Natriumsalz der Hyaluronsäure oder 3-Hydroxypropansäure und das Natriumsalz der Carboxymethylcellulose ein Puffersystem bilden.

10. Verfahren nach einem der Ansprüche 1 bis 5, **dadurch gekennzeichnet, dass** die gesamten Komponenten des Puffers in einer Konzentration von größer gleich 0,5 Gew.-% in der wässrigen Polysaccharidlösung gelöst sind.

11. Verfahren nach einem der Ansprüche 1 bis 8, **dadurch gekennzeichnet, dass** die Sterilisation der wässrigen Polysaccharidlösung in einem primären Packmittel erfolgt.

12. Verfahren nach Anspruch 9, **dadurch gekennzeichnet, dass** die Vermischung der wässrigen Polysaccharidlösung mit dem β-Lacton unmittelbar vor der Befüllung des primären Packmittels mit der wässrigen Polysaccharidlösung erfolgt.

13. Verfahren nach einem der Ansprüche 1 bis 9,
**gekennzeichnet, durch**
a) Lösen mindestens eines Polysaccharids in Wasser,
b) Zugabe von mindestens 0,5 Gew.-% β-Lacton zu der unter a) hergestellten wässrigen Polysaccharidlösung, und
c) Lagern des Gemisch bei einer Temperatur von 4°C bis 40 °C über einen Zeitraum von mindestens 24 Stunden, wobei
ein Puffersystem in der wässrigen Lösung enthalten ist, dessen Pufferkapazität so groß ist, dass die durch Hydrolyse des β-Lactons entstehende 3-Hydroxypropansäure so abgepuffert wird, dass der pH-Wert der sterilisierten wässrigen Polysaccharidlösung gleich der unsterilisierten Polysaccharidlösung ist.

14. Sterile wässrige Polysaccharidlösung hergestellt nach einem Verfahren der Ansprüche 1 bis 11, **dadurch gekennzeichnet, dass** die wässrige Polysaccharidlösung mindestens ein in Wasser zu mindestens teilweise lösliches Polysaccharid, Wasser und 3-Hydroxypropionsäure und ein Puffersystem enthält.

15. Sterile Polysaccharidlösung nach Anspruch 12, **dadurch gekennzeichnet, dass** Puffer-System Natrium-Ionen, Dihydrogenphosphat-Ionen, Hydrogenphosphatlonen, Phosphat-Ionen, 3-Hydroxypropionsäure und 3-Hydroxypropionat enthält.

16. Verwendung einer mit dem Verfahren nach den Ansprüchen 1 bis 11 hergestellten sterilen Polysaccharidlösung als Mittel zur Viscosupplementierung und als pharmazeutischer Wirkstoffträger in der Humanmedizin und in der Veterinärmedizin.

## Claims

1. Method for sterilisation of aqueous polysaccharide solutions,
**characterised in that**
a β-lactone is added to an aqueous solution of a polysaccharide in the presence of a buffer and the mixture is stored at a temperature of 4°C to 40°C for a period of time of at least 24 hours.

2. Method according to claim 1, **characterised in that**
the β-lactone is selected from compounds (a1), which are represented by general formula (I) 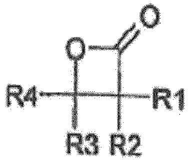 whereby R1, R2, R3, and R4 independently are H, a substituted or unsubstituted alkyl residue, a halide residue, a nitro residue or a cyano residue, and compounds (a2), which are selected from the group consisting of dimers of compounds (a1).

3. Method according to claim 2, **characterised in that**
the β-lactone is β-propiolactone (CAS number 57-57-8).

4. Method according to any one of the claims 1 to 3,
**characterised in that**
the amount of β-lactone that is added is more than/equal to 0.5% by weight, relative to the amount of the aqueous polysaccharide solution.

5. Method according to any one of the claims 1 to 4, **characterised in that** the polysaccharide is selected from the group consisting of the sodium salt of hyaluronic acid, the sodium salt of carboxymethylcellulose, hydroxyethyl cellulose, hydroxyethyl starch, methyl cellulose, and oxidised cellulose.

6. Method according to any one of the claims 1 to 5, **characterised in that** phosphate buffers with the pH value of 7.0-8.0, preferably with a pH value of 7.4, are used as buffer system.

7. Method according to claim 6, **characterised in that**
the phosphate buffer is selected from the group consisting of the combination of potassium hydrogen phosphate and disodium hydrogen phosphate, the combination of sodium hydrogen phosphate and disodium hydrogen phosphate, the combination of disodium phosphate and phosphoric acid, the combination of trisodium phosphate and sodium hydrogen phosphate, and the combination of trisodium phosphate and phosphoric acid.

8. Method according to claims 1 to 3, and 6, **characterised in that**
the 3-hydroxypropionic acid generated by the hydrolysis of the β-propiolactone is a component of the buffer system.

9. Method according to claims 1 to 3, **characterised in that** 3-hydroxypropanoic acid and the sodium salt of hyaluronic acid or 3-hydroxypropanoic acid and the sodium salt of carboxymethylcellulose form a buffer system.

10. Method according to any one of the claims 1 to 5, **characterised in that**
the total components of the buffer are dissolved in the aqueous polysaccharide solution at a concentration of more than/equal to 0.5% by weight.

11. Method according to any one of the claims 1 to 8, **characterised in that** the sterilisation of the aqueous polysaccharide solution takes place in a primary packaging means.

12. Method according to claim 9, **characterised in that** the mixing of the aqueous polysaccharide solution and the β-lactone takes place immediately before the aqueous polysaccharide solution is filled into the primary packaging means.

13. Method according to any one of the claims 1 to 9,
**characterised by**
a) dissolution of at least one polysaccharide in water;
b) addition of at least 0.5% by weight β-lactone to the aqueous polysaccharide solution prepared in a); and
c) storage of the mixture at a temperature of 4°C to 40°C for a period of time of at least 24 hours, whereby
the aqueous solution contains a buffer system whose buffering capacity is sufficiently such that the 3-hydroxypropanoic acid generated by the hydrolysis of the β-lactone is sufficiently buffered such that the pH value of the sterilised aqueous polysaccharide solution is equal to that of the non-sterilised polysaccharide solution.

14. Sterile aqueous polysaccharide solution prepared according to any one of the methods of claims 1 to 11, **characterised in that** the aqueous polysaccharide solution contains at least one polysaccharide that is at least partially soluble in water, water, and 3-hydroxypropanoic acid and a buffer system.

15. Sterile polysaccharide solution according to claim 12, **characterised in that** the buffer system contains sodium ions, dihydrogen phosphate ions, hydrogen phosphate ions, phosphate ions, 3-hydroxypropionic acid, and 3-hydroxypropionate.

16. Use of a sterile polysaccharide solution, which has been prepared by a method according to claims 1 to 11, as a means for visco-supplementation and as a pharmaceutical drug carrier in human medicine and veterinary medicine.

## Revendications

1. Procédé de stérilisation de solutions aqueuses de polysaccharide, **caractérisé en ce qu'**une β-lactone est ajoutée à une solution aqueuse d'un polysaccharide en présence d'un tampon et le mélange est stocké à une température de 4 °C à 40 °C pendant une période d'au moins 24 heures.

2. Procédé selon la revendication 1, **caractérisé en ce que** la β-lactone est sélectionnée parmi des composés (a1), qui sont représentés par la formule générale (I) 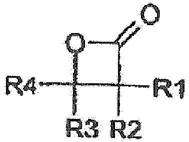 dans laquelle R₁, R2, R3 et R4 représentent indépendamment les uns des autres H, un radical alcoyle substitué ou non substitué, un radical halogène, un radical nitro ou un radical cyano, et des composés (a2) qui sont sélectionnés parmi le groupe qui se compose de dimères des composés (a1).

3. Procédé selon la revendication 2, **caractérisé en ce que** la β-lactone est de la β-propriolactone (numéro CAS 57-57-8).

4. Procédé selon une des revendications 1 à 3, **caractérisé en ce que** la β-lactone est ajoutée en une quantité supérieure ou égale à 0,5 % en poids, rapportée à la quantité de la solution aqueuse de polysaccharide.

5. Procédé selon une des revendications 1 à 4, **caractérisé en ce que** le polysaccharide est sélectionné parmi le groupe qui se compose du sel de sodium de l'acide hyaluronique, du sel de sodium de la carboxyméthylcellulose, de l'hydroxyéthylcellulose, de l'amidon hydroxyéthylique, de la méthylcellulose et de la cellulose oxydée.

6. Procédé selon une des revendications 1 à 5, **caractérisé en ce qu'**en tant que système de tampon, un tampon phosphate avec une valeur de pH de 7,0 à 8,0, de préférence avec une valeur de pH de 7,4, est utilisé.

7. Procédé selon la revendication 6, **caractérisé en ce que** le tampon phosphate est sélectionné parmi le groupe qui se compose de la combinaison d'hydrogénophosphate de potassium et d'hydrogénophosphate disodique, de la combinaison d'hydrogénophosphate de sodium et d'hydrogénophosphate disodique, de la combinaison de phosphate disodique et d'acide phosphorique, de la combinaison de phosphate trisodique et d'hydrogénophosphate de sodium et de la combinaison de phosphate trisodique et d'acide phosphorique.

8. Procédé selon les revendications 1 à 3 et 6, **caractérisé en ce que** l'acide 3-hydroxypropanoïque produit par l'hydrolyse de la β-propriolactone est un constituant du système de tampon.

9. Procédé selon les revendications 1 à 3, **caractérisé en ce que** l'acide 3-hydroxypropanoïque et le sel de sodium de l'acide hyaluronique ou l'acide 3-hydroxypropanoïque et le sel de sodium de la carboxyméthylcellulose forment un système de tampon.

10. Procédé selon une des revendications 1 à 5, **caractérisé en ce que** l'ensemble des composants du tampon sont dissous dans la solution aqueuse de polysaccharide dans une concentration supérieure ou égale à 0,5 % en poids.

11. Procédé selon une des revendications 1 à 8, **caractérisé en ce que** la stérilisation de la solution aqueuse de polysaccharide s'effectue dans un emballage primaire.

12. Procédé selon la revendication 9, **caractérisé en ce que** le mélange de la solution aqueuse de polysaccharide avec la β-lactone s'effectue juste avant le remplissage de l'emballage primaire avec la solution aqueuse de polysaccharide.

13. Procédé selon une des revendications 1 à 9,
**caractérisé par**
a) la dissolution d'au moins un polysaccharide dans de l'eau,
b) l'ajout d'au moins 0,5 % en poids de β-lactone à la solution aqueuse de polysaccharide produite en a), et
c) le stockage du mélange à une température de 4 °C à 40 °C pendant une période d'au moins 24 heures, dans lequel
un système de tampon est contenu dans la solution aqueuse dont la capacité de tampon est tellement grande que l'acide 3-hydroxypropanoïque produit par l'hydrolyse de la β-propriolactone est tamponné de sorte que la valeur de pH de la solution aqueuse de polysaccharide stérilisée est identique à la solution de polysaccharide non stérilisée.

14. Solution aqueuse de polysaccharide stérile, produite selon un procédé des revendications 1 à 11, **caractérisée en ce que** la solution aqueuse de polysaccharide contient au moins un polysaccharide au moins partiellement soluble dans l'eau, de l'eau et de l'acide 3-hydroxypropanoïque et un système de tampon.

15. Solution de polysaccharide stérile selon la revendication 12, **caractérisée en ce que** le système de tampon contient des ions sodium, des ions dihydrogénophosphate, des ions hydrogénophosphate, des ions phosphate, de l'acide 3-hydroxypropanoïque et du 3-hydroxypropionate.

16. Utilisation d'une solution de polysaccharide stérile, produite avec le procédé selon les revendications 1 à 11 en tant qu'agent pour la viscosupplémentation et en tant qu'excipient pharmaceutique dans la médecine humaine et dans la médecine vétérinaire.
